# EUROPÄISCHE PATENTSCHRIFT

(11) **EP 0 978 502 B1**
(45) Veröffentlichungstag und Bekanntmachung des Hinweises auf die Patenterteilung: **11.09.2002**
(21) Anmeldenummer: 99114328.0
(22) Anmeldetag: 21.07.1999
(51) Int. Cl.: C07C 67/03, C07C 69/716

(54) **Verfahren zur Umesterung von Alpha-Ketocarbonsäuren**
Process for the transesterification of alpha-ketocarboxylic acids
Procédé de transestérification d'acides alpha-cétocarboxyliques

(30) Priorität: 07.08.1998 AT 136598
(43) Veröffentlichungstag der Anmeldung: 09.02.2000
(73) Patentinhaber: DSM Fine Chemicals Austria Nfg GmbH & Co KG, 4021 Linz (AT)
(72) Erfinder: Zimmermann, Curt, 4310 Mauthausen (AT); Friedhuber, Johann, 4030 Linz (AT)
(74) Vertreter: Klostermann, Ingrid

(56) Entgegenhaltungen:
- DE-A- 2 319 478
- DE-A- 2 734 207
- DE-A- 3 334 208
- DE-C- 4 317 428
- US-A- 5 856 611
- T. MASUMIZU ET AL: TETRAHEDRON LETT., Bd. 27, Nr. 1, 1986, Seiten 55-56, XP002124385
- DATABASE CHEMABS [Online] CHEMICAL ABSTRACTS SERVICE, COLUMBUS, OHIO, US STN, CAPLUS accession no. 1978:616043, XP002124386 & JP 53 105417 A (MITSUBISHI RAYON CO LTD)

## Beschreibung

α-Ketocarbonsäureester, wie beispielsweise Brenztraubensäureethylester (PAEE), finden auf einer Vielzahl von Gebieten, wie etwa als Zwischenprodukte für Agro- und Pharmawirkstoffe, als Lösungsmittel u.s.w. Anwendung. Bisher angewandte Herstellungsverfahren erwiesen sich jedoch aus den unterschiedlichsten Gründen in wirtschaftlicher und technischer Hinsicht als ungeeignet. So ist beispielsweise die Herstellung von PAEE in Analogie zu Brenztraubensäuremethylester (PAME) durch Ozonolyse und anschließender Reduktion ausgehend von Ethylmethacrylat in Ethanol anstelle von Methylmethacrylat in Methanol mit wesentlichen Nachteilen behaftet. Erstens ist Ethylmethacrylat deutlich teurer als Methylmethacrylat, zweitens wird das Nebenprodukt Formaldehyd mit Ethanol als Lösungsmittel nicht vollständig acetalisiert, wodurch ein für die weitere Aufarbeitung störender Restgehalt an Formaldehyd verbleibt, drittens muß wegen des gebildeten schwereren Formaldehyddiethylacetals bei der Aufarbeitung mehr Nebenprodukt verbrannt werden und viertens die Ketalspaltung im Falle des PAEE-Diethylketals schwerer verläuft als beim PAME-Dimethylketal.

Gemäß J. Liebigs Ann. Chem., 564, 34 (1949) wird PAEE durch Veresterung von Brenztraubensäure mit abs. Ethanol und Benzol und anschließender Trocknung des temären Azeotrops Ethanol/Benzol/Wasser über K₂CO₃ in lediglich 53 %iger Ausbeute hergestellt.

Es wurden bereits Möglichkeiten untersucht PAEE durch Umesterung aus PAME herzustellen.
Dieser Herstellungsweg scheiterte bislang jedoch an der vielfältigen Reaktivität des Moleküls aufgrund des α-Ketocarbonsäurestrukturelementes. So kommt es bei der Umesterung im basischen Medium wegen einer raschen Kondensation der Carbonylgruppe mit der benachbarten, aktivierten Methyl- bzw. Methylengruppe zu unerwünschten Nebenprodukten, während im sauren Medium Ketale und Wasser gebildet werden, wobei das Wasser wiederum zur unerwünschten Hydrolyse des Esters führt. Unter annähernd neutralen Bedingungen werden keine akzeptablen Umsätze erzielt.
Aus der Literatur, beispielsweise aus DE 43 17 428, CAPLUS accession no. 1978:616043, XP002124386 & JP 53 105417, DE 33 34 208 oder US 5,856,611, sind bereits Umesterungsverfahren für weniger reaktive Ester, d.h. für Verbindungen, die in α-Stellung zur Carboxylgruppe, d.h. am benachbarten C-Atom, keine Oxogruppe aufweisen, wie etwa Acryl- und Methacrylsäureester oder β-Ketocarbonsäureester, bekannt.

In Tetrahedron Lett., Bd. 27, Nr. 1, 1986, Seiten 55-56 wird ein elektrokatalytisches Umesterungsverfahren u.a. von α-Ketocarbonsäureestern unter Verwendung von Lithiumperchlorat als Elektrolyt beschrieben, wobei Ausbeuten von max. 50% erzielt werden.

DE 23 19 478 beschreibt die Umesterung von Estern von Pregnansäure-Derivaten mit Alkali-, Erdalkali- oder Aluminiumalkoholaten, wobei gemäß Beispiel 2 Ausbeuten von höchstens 40% erzielt werden.

DE 27 34 207 beschreibt die Herstellung von α-Ketocarbonsäure-Derivaten, wie α-Ketocarbonsäureestern durch Oxidation aus den korrespondierenden α-Hydroxycarbonsäureestern.

Unerwarteterweise wurde nun gefunden, daß durch die Verwendung spezieller Metallkatalysatoren und Wasserfreiheit im Reaktionsmedium eine Umesterung von α-Ketocarbonsäureestem in guter Ausbeute und ohne Nebenreaktionen möglich ist.

Gegenstand der vorliegenden Erfindung ist demnach ein Verfahren zur Umesterung von α-Ketocarbonsäureestern mit einem Strukturelement der Formel I 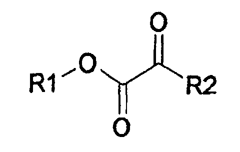 mit einem Alkohol der Formel (II) R₃OH,
wobei R₁, R₂ und R₃ einen verzweigten, unverzweigten oder cyclischen, gesättigten oder ungesättigten C₁ - C₃₀-Alkyl-, Aryl- oder Heteroarylrest bedeuten, und R₁ und R₃ nicht gleich sind, das dadurch gekennzeichnet ist, dass die Umesterung in einem wasserfreien Alkohol der Formel (II) als Reaktionsmedium unter Anwesenheit von Zinn- oder Titankatalysatoren bei einer Reaktionstemperatur von 20 bis 200°C unter Abdestillieren des abgespaltenen Alkohols durchgeführt wird.

Erfindungsgemäß können alle α-Ketocarbonsäureester mit dem Strukturelement der Formel I 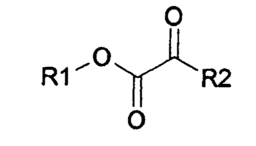 umgeestert werden.
R₁ und R₂ bedeuten dabei einen gesättigten oder ungesättigten, verzweigten, unverzweigten oder cyclischen C₁ - C₃₀-Alkylrest oder einen aromatischen oder heteroaromatischen Rest, wobei diese Reste Substituenten wie etwa C₁ - C₁₀-Alkoxy-,substituierten Amino-, Carbonyl-, derivatisierten Carboxyl- und underivatisierten Carboxyl-, Ester-, Halogen-, Hydroxy-, Nitro-Substituenten, sowie andere Stickstoff-Funktionen, Bor-, Phosphor-, Schwefel- oder Silizium-Verbindungen aufweisen können, wobei darauf zu achten ist, daß nicht neutral reagierende funktionelle Gruppen entweder intramolekular oder durch saure oder basische Zusätze bzw. Lösungsmittel weitgehend neutralisiert werden, um Kondensationsreaktionen bzw. sauer katalysierte Ketalierung zu verhindern.
Bevorzugt bedeuten R₁ und R₂ einen C₁- C₄-Alkylrest wie Methyl, Ethyl, Propyl- oder Isopropyl oder einen Benzylrest.
Besonders bevorzugt ist der Methylrest.

Das erfindungsgemäße Verfahren wird bevorzugt zur Umesterung von Brenztraubensäuremethylester (PAME) eingesetzt. Bevorzugtes Umesterungsprodukt ist Brenztraubensäureethylester (PAEE).
Die Umesterung erfolgt in einem wasserfreien Reaktionsmedium. Als Reaktionsmedium dient dabei ein Alkohol R₃-OH, wobei R₃ denjenigen Rest darstellt, der gegen R₁ ausgetauscht wird. R₃ ist demnach wie R₁ und R₂ definiert, wobei R₁ und R₃ nicht gleich sind.
Bevorzugt ist R₃ demnach ein verzweigter oder unverzweigter C₂- C₆-Alkylrest oder ein Benzylrest.
Bevorzugt wird nach dem erfindungsgemäßen Verfahren demnach PAME in wasserfreiem Ethanol zu PAEE umgesetzt.
Zusätzlich zum eingesetztem Alkohol kann noch ein weiteres wasserfreies Lösungsmittel, wie etwa unsubstituierte und substituierte Alkane, wie z.B. Hexan, Heptan, usw., Alkene, Alkine, Alkohole, substituierte Amine, Amide, Aromaten, Ester, Ether, Halogenverbindunge, Heteroaromaten, Lactone, Ketone, weitere stickstoffhältige Verbindungen wie z.B. Nitroalkane, Siliziumverbindungen wie z.B. Silikonöle, Schwefelverbindungen wie z.B. Sulfoxide verwendet werden, wobei wiederum darauf zu achten ist, daß nicht neutral reagierende funktionelle Gruppen entweder intramolekular oder durch saure oder basische Zusätze bzw. der Alkohol- oder EsterKomponente weitgehend neutralisiert werden, um Kondensationsreaktionen bzw. sauer katalysierte Ketalierung zu verhindern.

Die Umesterung findet erfindungsgemäß in Anwesenheit spezieller Metallkatalysatoren statt. Geeignete Katalysatoren sind
aus der Gruppe der Zinnkatalysatoren Dialkylzinndicarboxylate, wie Dibutylzinndicarboxylate insbesondere Dibutylzinndiacetat, Dibutylzinndilaurat, Dibutylzinndiisoctoat, Dibutylzinnmaleat und gemischte Dibutylzinndicarboxylate insbesondere mit längerkettigen Fettsäureresten, Dioctylzinndicarboxylate insbesondere Dioctylzinndilaurat, Trialkylzinnalkoxide, wie z. B. Tributylzinnoxid, Monoalkylzinnverbindungen wie Monobutylzinndihydroxychlorid und Monobutylzinndioxid, Zinnsalze wie Zinnacetat, Zinnoxalat, und Zinnchlorid, Zinnoxide, wie z. B. SnO,
sowie aus der Gruppe der Titankatalysatoren, monomere und polymere Titanate und Titanchelate wie Tetraisopropylorthotitanat, Tetrapropylorthotitanat, Tetraethylorthotitanat, Tetrabutylorthotitanat, Tetraisobutylorthotitanat, 2-Ethylhexyltitanat, Stearyltitanat, Kresyltitanat, Titanacetylacetonat, Triethanolamintitanat, Octylenglykoltitanat, Isostearyltitanat, Zitronensäurediethylestertitanat.

Bevorzugt werden Dibutylzinndiacetat, gemischte Dibutylzinndicarboxylate mit längerkettigen Fettsäureestern, Dioctylzinndilaurat, Monobutylzinndi-hydroxychlorid, Monobutylzinndioxid, Zinnacetat, Zinnoxalat, Zinnchlorid, Tetraisopropylorthotitanat, Tetrapropylorthotitanat, Tetraethylorthotitanat oder Tetra-butylorthotitanat eingesetzt.
Besonders bevorzugt sind Dibutylzinndiacetat, Tetraisopropylorthotitanat, Tetraethylorthotitanat.

Die Menge an eingesetztem Katalysator liegt bei 0,0001 bis 20 Gew.%, bevorzugt bei 0,005 bis 5 Gew.% und besonders bevorzugt bei 0,02 bis 1 Gew.%.

Das Reaktionsgemisch wird bevorzugt bis zum Siedepunkt des Reaktionsgemisches erhitzt, sodass die Reaktionstemperatur in Abhängigkeit von den Reaktanten zwischen 20°C und 200°C liegt. Die Umesterung kann weiters bei Normaldruck, aber auch bei reduziertem oder Über-Druck von 0,001 bis 200 bar durchgeführt werden. Der bei der Umesterung abgespaltene Alkohol wird bevorzugt kontinuierlich abdestilliert. Bevorzugt wird die Reaktion mit Hilfe einer Destillationskolonne mit hoher Trennleistung durchgeführt.

Die Abtrennung des Katalysators nach erfolgter Umsetzung gelingt in guter Ausbeute durch Waschen mit Wasser, Hydrolyse des Katalysators und Filtration des ausgefallenen Metalloxides oder bevorzugt durch Abdestillieren des Produktes vom Katalysator, vorzugsweise auf einem Dünnschicht- oder Kurzwegverdampfer.

Mit dem erfindungsgemäßen Verfahren werden Umsätze bis über 99 % erzielt, die Ausbeuten liegen ohne Rückführung von nicht umgesetzten Ausgangsprodukt bis über 96 %. Durch Rückführung des unumgesetzten Eduktes lassen sich Ausbeuten von bis über 97 % erzielen. Durch die schonenden Umesterungsbedingungen werden Produktreinheiten von bis über 99,9 % erhalten.

### Beispiel 1:

### Umesterung von PAME zu PAEE mit Dibutylzinndiacetat ohne Rückführung von PAME

800 g /7,8 mol) Brenztraubensäuremethylester (99,8 % GC) und 900 g Ethanol (wasserfrei) wurden in einer Destillationsapparatur mit einer 25-bödigen Glockenbodenkolonne und Rücklaufteiler zum Sieden erhitzt. Das Destillat am Kopf der Kolonne wurde bei 100 % Rücklauf auf Wasserfreiheit überprüft (< 0,1 % Wasser nach Karl Fischer). Danach wurden 0,31 g Dibutylzinndiacetat in Form einer 10 % Lösung in Ethanol zugegeben. Es wurde noch 2 Stunden auf 100 % Rücklauf Methanol, welches durch die Umesterunsreaktion entsteht, am Kopf der Kolonnen gesammelt und anschließend bei einem Verhältnis Rücklauf zu Abnahme von 20 : 1 abdestilliert. Der Füllstand im Reaktionsgefäß wurde durch Zugabe von Ethanol in den Destillationssumpf konstant gehalten. Nach 12 Stunden war die Reaktion praktisch beendet. Dies war aus dem Abklingen der sich am Kopf der Destillationskolonne ansammelnden Methanolmenge leicht erkennbar. Überschüssiges Ethanol wurde nun bei vermindertem Druck abdestilliert, wobei die Sumpftemperatur nicht über ca. 90°C anstieg. Die verbleibende Reaktionslösung wurde auf einem Dünnschichtverdampfer unter vermindertem Druck von 80mbar vom Zinnkatalysator befreit (30 g zinnhaltiger Destillationssumpf). Durch fraktionierte Destillation bei 50 mbar wurden 880 g (7,6 mol) Brenztraubensäureethylester mit einer Reinheit von 99,8 % (GC) erhalten.
Die Ausbeute betrug 96,7 %.

In einem Vergleichsversuch mit Ethanol, welches einen Wassergehalt von 0,6 % (KF) aufwies, wurde nach 12 Stunden Reaktionszeit durch GC-MS Analyse neben dem gewünschten Produkt die Dimethyl-, Diethyl- und gemischten Ethyl-Methyl-Ketale des Brenztraubensäuremethylesters und Brenztraubensäureethylesters gefunden. Bei der Aufarbeitung fielen 155 g nicht mehr weiter verwertbarer Dünnschicht-Destillations-Sumpf an. Nach fraktionierter Destillation wurden 755 g (6,3 mol) Brenztraubensäureethylester mit einer Reinheit von 97,1 % (GC) erhalten. Die Ausbeute betrug 81 %.

### Beispiel 2:

### Umesterung von PAME zu PAEE mit Dibutylzinndiacetat mit Rückführung von PAME.

1000 g (9,8 mol) Brenztraubensäuremethylester, 1100 g Ethanol und 0,39 g Dibutylzinndiacetat wurden wie im Beispiel 1 umgesetzt. Nach 8 Stunden wurde die Reaktion abgebrochen. Das Reaktionsgemisch wurde wie im Beispiel 1 aufgearbeitet. Es verblieben 25 g zinnhaltiger Destillationssumpf. Durch fraktionierte Destillation wurden 201 g (2 mol) nicht umgesetzter Brenztraubensäuremethylester mit einer Reinheit von 98 % (GC), welcher als Einsatz für den nächsten Versuch diente, sowie 884 g (7,6 mol) Brenztraubensäureethylester mit einer Reinheit von 99,9 % (GC) erhalten. Die Ausbeute an Brenztraubensäureethylester bezogen auf umgesetzten Brenztraubensäuremethylester betrug 97,3 %.

### Beispiel 3:

### Kontinuierliche Umesterung von PAME zu PAEE mit Titan(IV)-isopropanolat

1000 g (9,8 mol) Brenztraubensäuremethylester und 20 g Titan(IV)-isopropanolat wurden in 750 g Ethanol gelöst. Im Sumpf einer Destillationsapparatur, bestehend aus Kolonne, Kondensator mit Rücklaufteiler, Gefäße für Reaktanten und Produkte sowie Pumpen zum ein- und austragen der Produktströme und zum evakuieren der Apparatur, wurden 300 ml Ethanol unter leichtem Vakuum (395 mbar) erhitzt. Danach wurden die Edukte mit konstanter Rate über einen Zeitraum von 5 Stunden im oberen Drittel der Kolonne eingetragen. Während dieser Zeit wurden weitere 790 g Ethanol kontinuierlich in den Sumpf der Kolonne eingebracht wobei die Sumpftemperatur bei 66°C gehalten wurde. Der Füllstand im Sumpfgefäß wurde durch kontinuierliche Austragung des Reaktionsgemisches bei ca. 300 ml gehalten. Am Kopf der Kolonne wurde das entstandene Methanol mit einem Rücklauf- zu Abnahmeverhältnis von 1 : 1 abdestilliert. Nachdem die Einbringung der Edukte beendet war, wurde das gesamte am Sumpf der Kolonne angefallene Reaktionsgemisch vereint und wie in Beispiel 2 beschrieben aufgearbeitet. Es verblieben 80 g titanhaltiger Dünnschicht-Destillations-Sumpf. Die fraktionierte Destillation ergab 158 g (1,55 mol) an nicht umgesetzten Brenztraubensäuremethylester, sowie 899 g (7,7 mol) Brenztraubensäuremethylester mit einer Reinheit von 99,7 % (GC). Die Ausbeute an Brenztraubensäureethylester bezogen auf umgesetzten Brenztraubensäuremethylester betrug 94 %.

## Patentansprüche

1. Verfahren zur Umesterung von α-Ketocarbonsäureestern mit einem Strukturelement der Formel I 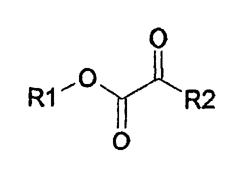 mit einem Alkohol der Formel (II) R₃OH,
wobei R₁, R₂ und R₃ einen verzweigten, unverzweigten oder cyclischen, gesättigten oder ungesättigten C₁ - C₃₀-Alkyl-, Aryl- oder Heteroarylrest bedeuten, und R₁ und R₃ nicht gleich sind, **dadurch gekennzeichnet, dass** die Umesterung in einem wasserfreien Alkohol der Formel (II) als Reaktionsmedium unter Anwesenheit von Zinn- oder Titankatalysatoren bei einer Reaktionstemperatur von 20 bis 200°C unter Abdestillieren des abgespaltenen Alkohols durchgeführt wird.

2. Verfahren nach Anspruch 1, **dadurch gekennzeichnet, dass** Ketocarbonsäureester der Formel I in der R₁ und R₂ einen verzweigten oder unverzweigten C₁-C₄-Alkylrest oder einen Benzylrest bedeuten mit einem Alkohol R₃OH umgeestert werden, wobei R₃ nicht gleich R₁ ist und einen verzweigten oder unverzweigten C₂ bis C₆-Alkylrest oder ein Benzylrest bedeutet.

3. Verfahren nach Anspruch 1, **dadurch gekennzeichnet, dass** Brenztraubensäuremethylester in wasserfreiem Ethanol zu Brenztraubensäureethylester umgeestert wird.

4. Verfahren nach Anspruch 1, **dadurch gekennzeichnet, dass** der Katalysator in einer Menge von 0,0001 bis 20 Gew.%, bevorzugt von 0,005 bis 5 Gew.% und besonders bevorzugt von 0,02 bis 1 Gew.% eingesetzt wird.

5. Verfahren nach Anspruch 1, **dadurch gekennzeichnet, dass** als Katalysator aus der Gruppe der Zinnkatalysatoren Dibutylzinndiacetat, -dilauraut, -diisooctoat oder -maleat, gemischte Dibutylzinndicarboxylate mit längerkettigen Fettsäureestern, Dioctylzinndilaurat, Monobutylzinndihydroxychlorid, Monobutylzinndioxid, Zinnacetat, Zinnoxalat oder Zinnchlorid eingesetzt wird.

6. Verfahren nach Anpruch 1, **dadurch gekennzeichnet, dass** als Katalysator aus der Gruppe der Titankatalysatoren Tetraisopropyl-, Tetrapropyl-, Tetraethyl- oder Tetrabutylorthotitanat eingesetzt wird.

7. Verfahren nach Anspruch 1, **dadurch gekennzeichnet, dass** die Umesterung in Anwesenheit eines zusätzlichen Lösungsmittels durchgeführt wird.

## Claims

1. A process for transesterifying α-ketocarboxylic esters having a structural element of the formula I 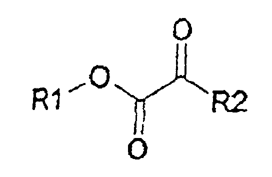 using an alcohol of the formula (II) R₃OH,
where R₁, R₂ and R₃ are a branched, unbranched or cyclic, saturated or unsaturated C₁-C₃₀ alkyl, aryl or heteroaryl radical, and R₁ and R₃ are not identical, which comprises carrying out the transesterification in an anhydrous alcohol of the formula (II) as reaction medium in the presence of tin catalysts or titanium catalysts at a reaction temperature of 20 to 200°C, distilling off the eliminated alcohol.

2. The process as claimed in claim 1, wherein ketocarboxylic esters of the formula I where R₁ and R₂ are a branched or unbranched C₁-C₄ alkyl radical or a benzyl radical are transesterified with an alcohol R₃OH, where R₃ is not identical to R₁ and is a branched or unbranched C₂ to C₆ alkyl radical or a benzyl radical.

3. The process as claimed in claim 1, wherein methyl pyruvate is transesterified in anhydrous ethanol to form ethyl pyruvate.

4. The process as claimed in claim 1, wherein the catalyst is used in an amount from 0.0001 to 20% by weight, preferably from 0.005 to 5% by weight, and particularly preferably from 0.02 to 1% by weight.

5. The process as claimed in claim 1, wherein the catalyst used is selected from the group consisting of the tin catalysts dibutyltin diacetate, dibutyltin dilaurate, dibutyltin diisooctoate or dibutyltin maleate, mixed dibutyltin dicarboxylates with relatively long-chain fatty esters, dioctyltin dilaurate, monobutyltin dihydroxychloride, monobutyltin dioxide, tin acetate, tin oxalate or tin chloride.

6. The process as claimed in claim 1, wherein the catalyst used is selected from the group consisting of the titanium catalysts tetraisopropylorthotitanate, tetrapropylorthotitanate, tetraethylorthotitanate or tetrabutylorthotitanate.

7. The process as claimed in claim 1, wherein the transesterification is carried out in the presence of an additional solvent.

## Revendications

1. Procédé de transestérification d'esters d'acides α-cétocarboxyliques avec un élément structurel de formule I avec un alcool de formule (II), R₃OH,
dans lesquelles R₁, R₂ et R₃ signifient un reste alkyle en C₁ - C₃₀, aryle ou hétéroaryle, ramifié, non ramifié ou cyclique, saturé ou insaturé, et R₁ et R₃ ne sont pas identiques, **caractérisé en ce qu'**on effectue la transestérification dans un alcool anhydre de formule (II) en tant que milieu de réaction, en présence de catalyseurs à base d'étain ou de titane, à une température de réaction de 20 à 200°C, avec élimination par distillation de l'alcool séparé.

2. Procédé selon la revendication 1, **caractérisé en ce que** les esters d'acides cétocarboxyliques de formule I, dans laquelle R₁ et R₂ signifient un reste alkyle en C₁ - C₄, ramifié ou non ramifié, ou un reste benzyle, sont transestérifiés avec un alcool R₃OH, R₃ n'étant pas identique à R₁ et signifiant un reste alkyle en C₂ à C₆, ramifié ou non ramifié, ou un reste benzyle.

3. Procédé selon la revendication 1, **caractérisé en ce que** l'ester méthylique d'acide pyruvique est transestérifié en ester éthylique d'acide pyruvique dans de l'éthanol anhydre.

4. Procédé selon la revendication 1, **caractérisé en ce qu'**on utilise le catalyseur en une quantité de 0,0001 à 20 % en poids, de préférence de 0,005 à 5 % en poids et de façon particulièrement préférée de 0,02 à 1 % en poids.

5. Procédé selon la revendication 1, **caractérisé en ce qu'**on utilise un catalyseur choisi dans l'ensemble des catalyseurs à l'étain, diacétate, dilaurate, diisooctoate ou diisomaléate de dibutylétain, des dicarboxylates mélangés de dibutylétain avec des esters d'acides à chaîne longue, le dilaurate de dioctylétain, le dihydroxychlorure de monobutylétain, le dioxyde de monobutylétain, l'acétate d'étain, l'oxalate d'étain ou le chlorure d'étain.

6. Procédé selon la revendication 1, **caractérisé en ce qu'**on utilise un catalyseur choisi dans l'ensemble des catalyseurs au titane que sont les orthotitanates de tétraisopropyle, tétrapropyle, tétraéthyle ou tétrabutyle.

7. Procédé selon la revendication 1, **caractérisé en ce qu'**on effectue la transestérification en présence d'un solvant supplémentaire.
